(19) **Europäisches Patentamt**
**European Patent Office**
**Office européen des brevets**

(11) **EP 1 752 544 B1**

(12) **EUROPEAN PATENT SPECIFICATION**

(45) Date of publication and mention
of the grant of the patent:
**21.08.2013 Bulletin 2013/34**

(51) Int Cl.:
*G01N 33/566* (2006.01)   *G01N 21/78* (2006.01)

(21) Application number: **05710442.4**

(22) Date of filing: **14.02.2005**

(86) International application number:
**PCT/JP2005/002660**

(87) International publication number:
**WO 2005/078119 (25.08.2005 Gazette 2005/34)**

(54) **PROBE FOR DETECTING NUCLEAR RECEPTOR AGONIST OR ANTAGONIST AND METHOD OF SCREENING AGONIST OR ANTAGONIST TO NUCLEAR RECEPTOR WITH THE USE OF THE SAME**

SONDE ZUM NACHWEIS EINES AGONISTEN ODER ANTAGONISTEN EINES NUKLEÄREN REZEPTORS SOWIE VERFAHREN ZUM SCREENING EINES AGONISTEN ODER ANTAGONISTEN ZU EINEM NUKLEÄREN REZEPTOR UNTER VERWENDUNG DAVON

SONDE SERVANT A DETECTER UN AGONISTE OU ANTAGONISTE DE RECEPTEUR NUCLEAIRE ET PROCEDE DE RECHERCHE PAR CRIBLAGE D'UN AGONISTE OU ANTAGONISTE VIS-A-VIS D'UN RECEPTEUR NUCLEAIRE EN UTILISANT CELLE-CI

(84) Designated Contracting States:
**CH DE FR GB LI NL SE**

(30) Priority: **12.02.2004 JP 2004035678**

(43) Date of publication of application:
**14.02.2007 Bulletin 2007/07**

(73) Proprietor: **Japan Science and Technology Agency**
**Kawaguchi-shi,**
**Saitama 332-0012 (JP)**

(72) Inventors:
• **UMEZAWA, Yoshio**
**Shinjuku-ku, Tokyo 162-0063 (JP)**
• **SATO, Moritoshi**
**Bunkyo-ku, Tokyo 113-0023 (JP)**

(74) Representative: **Owen, Deborah Jane**
**Dehns**
**St Bride's House**
**10 Salisbury Square**
**London**
**EC4Y 8JD (GB)**

(56) References cited:
EP-A- 1 229 330    WO-A1-02/08766
JP-A- 2001 183 366

• LLOPIS J ET AL: "LIGAND-DEPENDENT INTERACTIONS OF COACTIVATORS STEROID RECEPTOR COACTIVATOR-1 AND PEROXISOME PROLIFERATOR-ACTIVATED RECEPTOR BINDING PROTEIN WITH NUCLEAR HORMONE RECEPTORS CAN BE IMAGED IN LIVE CELLS AND ARE REQUIRED FOR TRANSCRIPTION" PROCEEDINGS OF THE NATIONAL ACADEMY OF SCIENCES OF USA, NATIONAL ACADEMY OF SCIENCE, WASHINGTON, DC, US, vol. 97, no. 8, 11 April 2000 (2000-04-11), pages 4363-4368, XP001035161 ISSN: 0027-8424
• AWAIS M ET AL: "A GENETICALLY ENCODED FLUORESCENT INDICATOR CAPABLE OF DISCRIMINATING ESTROGEN AGONISTS FROM ANTAGONISTS IN LIVING CELLS" ANALYTICAL CHEMISTRY, AMERICAN CHEMICAL SOCIETY. COLUMBUS, US, vol. 76, no. 8, 15 April 2004 (2004-04-15), pages 2181-2186, XP001196787 ISSN: 0003-2700
• KAWAI Y ET AL: "Single color fluorescent indicators of protein phosphorylation for multicolor imaging of intracellular signal flow dynamics" ANALYTICAL CHEMISTRY, AMERICAN CHEMICAL SOCIETY. COLUMBUS, US, vol. 76, no. 20, 15 September 2004 (2004-09-15), pages 6144-6149, XP002368363 ISSN: 0003-2700

EP 1 752 544 B1

- **SATO M. ET AL - NLM11875431 MEDLINE: 'Fluorescent indicators for imaging protein phosphorilation in single living cells.' NAT.BIOTECHNOL. vol. 20, no. 3, 2002, pages 287 - 294, XP002982401**
- **CALLEJA V. ET AL: 'Monitoring conformational changes of proteins in cells by fluorescence lifetime imaging microscopy.' BIOCHEM. J. vol. 372, 2003, pages 33 - 40, XP002327254**
- **SASAKI K. ET AL: 'Fluorescent indicators for Akt/ protein kinase B and dynamics of Akt activity visualized in living cells.' J.BIOL.CHEM. vol. 278, no. 33, 2003, pages 30945 - 30951, XP002989163**
- **UMEZAWA Y. ET AL: 'Naibunpitsu Kakuran Kagaku Busshitsu no Saibonai Hyoteki Bunshi no Dotei to Atarashii Biomonitoring.' January 2004, pages 1 - 11, XP002991299**

## Description

Probe for Detecting Nuclear Receptor Agonist or Antagonist and Method for Screening Agonist or Antagonist to Nuclear Receptor using the Same

## Technical Field

The invention of this application relates to a probe for detecting an agonist or an antagonist to a nuclear receptor. More particularly, the invention relates to a probe for detecting and quantifying an agonist or an antagonist to a nuclear receptor with a high selectivity, and also to a method for screening an agonist and/or an antagonist to a nuclear receptor using the same.

## Background Art

Lipophilic signal molecules such as steroid hormones including estrogen, progesterone, androgen, glucocorticoid and mineralocorticoid, thyroid gland hormones and retinoic acids may be deeply participated in life activities of higher animals such as regulation of proliferation and differentiation of target tissues (cells), ontogeny and spiritual activity, as well as in onset and development of many diseases. Receptors specifically bind to such lipophilic signal molecules exist in cytosol and nucleus, which are so called as "nuclear receptors" and form a gene superfamily.

Such nuclear receptors are known as a ligand-dependent transcription factor that binds to responsive element on a promoter of target gene upon specifically binding to the lipophilic signal molecules, thereby promoting the transcription.

Recent studies on nuclear receptors also show that in the transcription regulation mechanism of nuclear receptors, not only a ligand but also a coactivator positively or negatively regulating the transcription plays an important role.

For example, estrogen is involved in growth, development and maintenance of germ cells (Non-Patent Document 1), and exerts physiological and pharmacological effects upon binding to an estrogen receptor (ER) (Non-Patent Documents 2 and 3). Further, steroid receptor coactivator 1 (SRC-1) belonging to p160 nuclear hormone receptor coactivator family interacts with a coactivator-binding site of the ER and induces a transcription activity of the ER when it forms a complex with an agonist, but not when an agonist is absent or the ER binds to an antagonist (Non-Patent Document 4). That is, the agonist induces conformational change of the ER to form a coactivator-binding site on the ER thereby binding of the coactivator to the ER is promoted and stabilized. Also, as a result of binding of the ER to the coactivator, binding of the ER to the agonist is also mutually stabilized and a dissociation rate of the agonist from the ER is significantly lowered (Non-Patent Document 5).

Many synthetic chemicals having little structural resemblance to the natural estrogens may mimic or block the natural estrogen activities in a living body by binding to the ER. These chemicals are called endocrine disruptors (Non-Patent Documents 6 to 9). Early developmental exposure to these estrogenic chemicals is known to cause abnormality or weight loss of germ organs, or qualitative and quantitative decrease of sperms (Non-Patent Documents 10 to 12). The endocrine disruptors for lipophilic signal molecules other than estrogens have been also clarified.

Therefore, many studies have been carried out for testing a binding activity of a test compound to ER (Non-Patent Documents 7 and 13 to 16). All of those conventional tests are based on a competitive reaction in which a test compound displaces a labeled ligand, usually a radioactive 17β-estradiol (E2), which is bound to ER. Large-scale screening of endocrine disruptors is possible by the conventional methods, but a number of shortcomings are there: for example, it is not possible to distinguish between agonistic and antagonistic effects, as the binding of antagonists to the receptor do not result in transcriptional activation

These conventional methods are not suitable to find the binding affinities of the compounds having low aqueous solubility. Moreover, incubation is necessary at subphysiological temperature; several washes are required to separate the free radiolabeled molecule from the molecules bound to ER before measurement, which may disturb the reaction equilibrium between ER and a ligand.

Recently, new in vitro binding assays have been proposed using no radioisotopes, which include a fluorescence polarization binding assay (Non-Patent Document 17), an electrochemical binding assay (Non-Patent Document 18) and a surface plasmon resonance biosensor technique (Non-Patent Document 19). However, they are also unable to distinguish between an agonist and an antagonist of estrogen. Moreover, a receptor-binding assay requires a large amount of pure receptor proteins.

Further, E-screening using MCF-7 cells or T47D cells is employed for a cell growth assay, which analyzes the growth stimulating ability of a chemical to estrogen-sensitive cells (Non-Patent Document 20). Moreover, the reporter gene assay in yeast and mammalian cells, an analysis of the ability of a chemical to stimulate the transcription of a reporter gene construct in a cell culture, is very useful and powerful tools to identify substances of estrogenic potency (Non-Patent Documents 21 and 22). Although the E-screening and reporter gene assay have the ability to distinguish between agonists and antagonists, one has to wait for a period of almost one day to get the final results after a ligand is added in the culture media containing mammalian cells or yeast.

Llopis J. et al. (2000) PNAS 97(8): 4363-4368 discloses that the fluorescently-tagged nuclear receptors ER (estrogen receptor) and RAR (retinoic acid receptor) exhibit ligand-dependent fluorescence resonance energy transfer (FRET) to fluorescently-tagged nuclear receptor interaction domains of the coactivators SRC-1 (steroid receptor coactivator-1) and PBP (peroxisome proliferator binding protein). The inhibitors of FRET by tamoxifen, and the reversal of said inhibitors by estradiol, is also disclosed. The association of the tagged nuclear receptors and tagged coactivators was observed in living cells.

Accordingly, there are no simple and convenient method for screening an agonist and an antagonist to nuclear receptors such as estrogen with high speed and high selectivity.

In view of the aforementioned circumstances, a purpose of the present invention is to provide a method for screening an agonist and an antagonist to nuclear receptors with high selectivity.

**Patent Documents**

    1: Japanese Patent Application No. 2003-145466
    2: Japanese Patent Application No. 2003-301259

**Non-Patent Document**

    1: Ciocca, D. R. and Roig, L. M. V. Endocr. Rev. 1995, 16, 35-62.
    2: Tsai, M. J., and O'Malley, B. W. Annu. Rev. Biochem. 1994, 63, 451-486.
    3: Nilsson, S. et al. Physiological Rev. 2001, 81, 1535-1565.
    4: McKenna, N. J., et al. Endocr. Rev. 1999, 20, 321-344.
    5: Gee, A. C., et al. Mol. Endocrinol. 1999, 13, 1912-1923.
    6: Korach, K. S. Endocrinology 1993, 132, 2277-2278.
    7: Shelby, M. D., et al. Environ. Health Perspect. 1996, 104, 1296-1300.
    8: Oosterkamp, A. J., et al. Anal. Chem. 1997, 16, 545-553.
    9: Sonnenschein, C.; Soto, A. M. J. Steroid Biochem. Mol. Biol. 1998, 65, 143-150.
    10: Colborn T. Environ. Health Perspect. 1995, 103, 135-136.
    11: Neubert, D., Regul. Toxicol. Pharmacol. 1997, 26, 9-29.
    12: Daston, G. P., et al. J. W. Reprod. Toxicol. 1997, 11, 465-481.
    13: Salomonsson, M., et al. J. Steroid Biochem. Mol. Biol. 1994, 50, 313-318.
    14: Kuiper, G. G. J. M., et al. Endocrinology 1997, 138, 863-870.
    15: Tabira, et al. Eur. J. Biochem. 1999, 262, 240-245.
    16: Blair, R. M., et al. Toxicol. Sci. 2000, 54, 138-155.
    17: Bolger, R., et al. Environ. Health Perspect. 1998, 106, 551-557.
    18: Kuramitz, H., et al. Anal. Chem. 2002, 74, 533-538.
    19: Usami, M., et al. Steroid Biochem. Mol. Biol. 2002. 81, 47-55.
    20: Soto, A. M., et al. Environ. Health Perspect. 1995. 103 (Suppl. 7), 113-122.
    21: Bronstein, I., et al. J. Anal. Biochem. 1994, 219, 169-181.
    22: Gaido, K. W. et al. Toxicol. Appl. Pharmcol. 1997, 143, 205-213.
    23: Herry, D. M., et al. Nature 1997, 387, 733-736.
    24: Mak, H. Y., et al. Mol. Cell. Biol. 1999. 19, 3895-3903.
    25: Weatherman, R. V., et al. Mol Endocrinol. 2002, 16, 487-496.
    26: Ueda, H., et al. J. Immunol. Methods. 2003, 279(1-2), 209-18.
    27: Pollock, B. A.; Heim, R. Cell Biol. 1999, 9, 57-60.
    28: Mochizuki, N., et al. Nature 2001. 411, 1065-1068.
    29: Sato M., et al. Nature Biotech. 2002, 20, 287-294.
    30: Sasaki, K., et al. Biol. Chem. 2003, 278, 30945-30951.
    31: Proc. Natl. Acad. Sci. USA 77: 7380-7384, 1980
    32: Brzozowski, A. M., et al. Nature 1997, 389. 753-758.
    33: Shiau, A. K., et al. Cell 1998, 95, 927-937.
    34: Routledge, E. J., et al. J. Biol. Chem. 2000, 275, 35986-35993.

35: Fang, H., et al. Chem. Res. Toxicol. 2001, 14, 280-294.
36: Jordan, V. C., et al. Cancer Res. 2001, 61, 6619-6623.

## Disclosure of the Invention

In order to solve the aforementioned problems, the present invention firstly provides a probe for detecting an agonist or an antagonist to a nuclear receptor, in which, at least, a ligand-recognition site containing a ligand-binding domain of the nuclear receptor is connected with a binding-responsive site containing a peptide chain that specifically binds to a coactivator-binding site in the ligand-binding domain by a flexible linker to construct a fusion structure [ligand-recognition site/ linker/binding-responsive site], and two reporters are connected with the respective ends of the fusion structure.

Secondly, the present invention provides the probe in which the ligand-recognition site contains a ligand-binding domain of a nuclear receptor selected from the group including glucocorticoid receptor, estrogen receptor, progesterone receptor, peroxisome proliferator-activated receptor, androgen receptor, thyroid gland hormone receptor, retinoic acid receptor, vitamin D receptor and an orphan receptors. Thirdly, it provides the probe in which the ligand-recognition site is an estrogen receptor $\alpha$ ligand-binding domain, a peroxisome proliferator-activated receptor ligand-binding domain or an androgen receptor ligand-binding domain.

Fourthly, the invention of this application provides the probe in which the binding-responsive site is a nuclear receptor interaction domain peptide of a steroid receptor coactivator 1. Fifthly, it provides the probe in which the binding-responsive site contains the motif of SEQ ID No: 1. Sixthly, it provides the probe in which the two reporters are of a yellow fluorescent protein and a cyan fluorescent protein.

Seventhly, the present invention provides a method for screening nuclear receptor agonists, which comprises making any of the aforementioned probes coexist with an agonist candidate substance, and measuring changes in signals with and without the agonist candidate substance. Eighthly, it provides the method in which the probe coexists with the agonist candidate substance in cells by introducing a polynucleotide expressing the probe into the cells. Ninthly, it provides the method in which the probe coexists with the agonist candidate substance in all cells of a non-human animal or its progeny by introducing a polynucleotide expressing the probe into a non-human animal totipotent cell and developing the cell into a individual animal.

Tenthly, the present invention provides a method for screening nuclear receptor antagonists, which comprises making any of the aforementioned probes coexist with an excessive amount of antagonist candidate substance and a known agonist, and measuring changes in a signal with and without antagonist candidate substances. Eleventhly, it provides the method in which the probe coexists with the agonist and antagonist candidate substance in cells by introducing a polynucleotide expressing the probe into the cells. Twelfthly, it provides the method in which the probe coexists with the agonist and antagonist candidate substance in all cells of a non-human animal or its progeny by introducing a polynucleotide expressing the probe into a non-human animal totipotent cell and developing the cell into an individual animal.

Thirteenthly, the invention of this application also provides a non-human animal or its progeny, which is established by introducing a polynucleotide expressing any of the aforementioned probes into non-human animal totipotent cell and developing the cell into an individual animal.

## Brief Description of Drawings

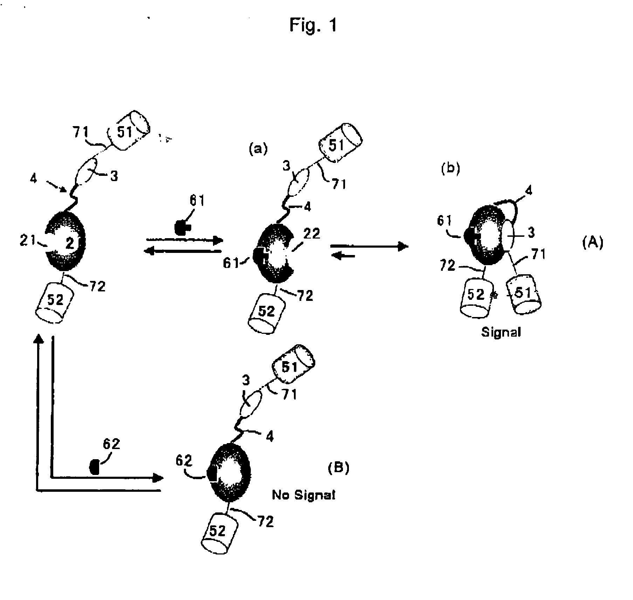

Fig. 1 is a brief schematic depiction which exemplifies the constitution and the operation of the probe for detecting an agonist or antagonist according to this invention.

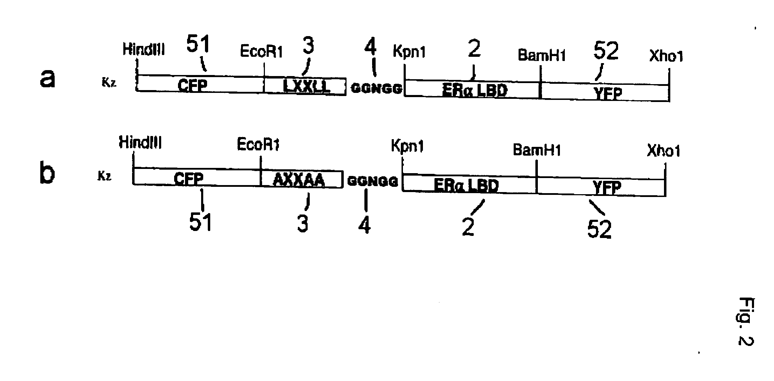

Fig. 2A is a brief schematic depiction which shows the constitution of a probe for detecting an estrogen agonist or antagonist to an estrogen receptor (a) and a mutant probe (b); Fig. 2B is a brief schematic depiction which shows the constitution of a probe for detecting an agonist or antagonist to a peroxisome proliferator-activated receptor and a mutant probe; and Fig. 2C is a brief schematic depiction which shows the constitution of a probe for detecting an agonist or antagonist to an androgen receptor (a) and a mutant probe (b).

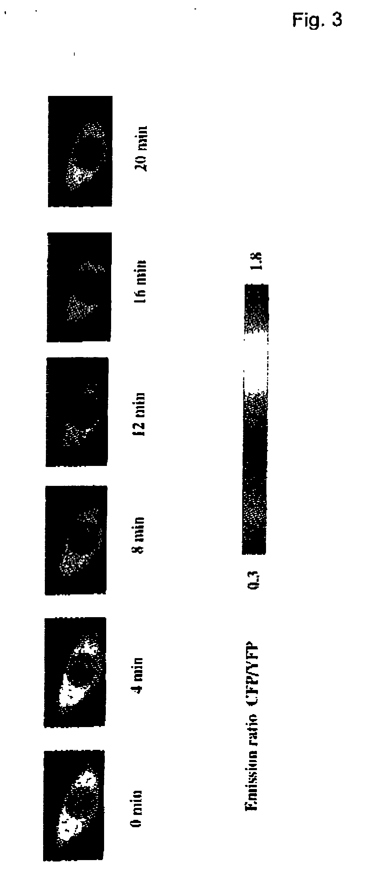

Fig. 3A is a set of pictures of cells showing the time course in FRET when the probe-expressing CHO-K1 is stimulated with 100 nM E2; and Fig. 3B is a set of pictures of cells showing the time course in FRET when PK-15 cell is stimulated with 100 nM DHT. The fluorescence intensity ratio of 480/535 nm is shown as a pseudo-color picture.

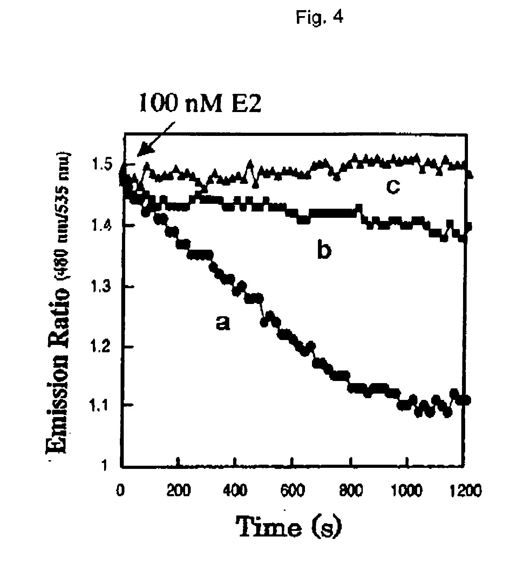

Fig. 4A is a time course in FRET when the probe-expressing CHO-K1 cell is stimulated with 100 nM E2 in the Example of this invention (a: after addition of E2; b: no E2 was added; c: the mutant probe-expressing CHO-K1 cell stimulated with E2); Fig. 4B is a time course in FRET when the probe-expressing CHO-K1 cell is stimulated with 15d-PGJ$_2$ in the Example of this invention (a: time course; b: relation (response curve) between concentration of 15d-PGJ$_2$ and values of changes in fluorescence intensity ratio); Fig. 4C is a time course in FRET when the probe-expressing CHO-K1 cell is stimulated with pioglitazone in the Example of this invention (a: time course; b: relation (response curve) between concentration of pioglitazone and values of changes in fluorescence intensity ratio); and Fig. 4D is a time course in FRET when the probe-expressing CHO-K1 cell is stimulated with 100 nM DHT in the Example of this invention (a: after addition of DHT; b: no DHT was added; c: the mutant probe-expressing PK-15 cell stimulated with DHT).

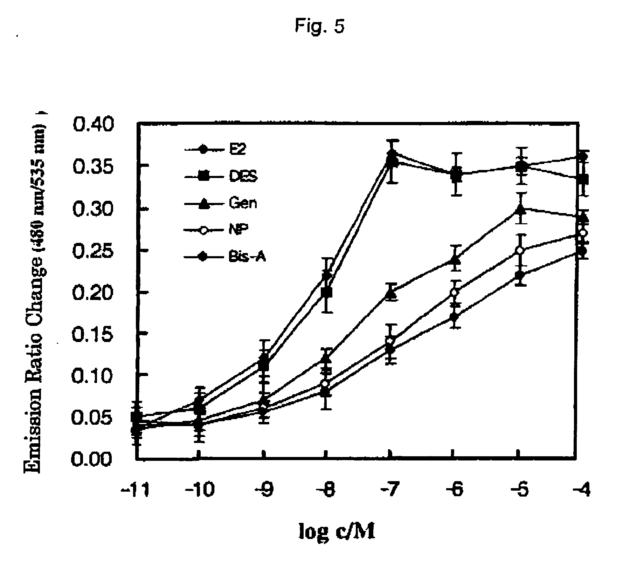

Fig. 5A shows the relation (response curve) between concentration and values of changes in fluorescence intensity ratio when, in the Example of this invention, a probe is expressed in CHO-K1 cell and exogenous estrogen such as DES. Gen, Bis-A or NP is added; and Fig. 5B shows the relation (response curve) between concentration and values of changes in fluorescence intensity ratio when, in the Example of this invention, a probe is expressed in PK-15 cell and then DHT, testosterone, progesterone or cortisol is added.

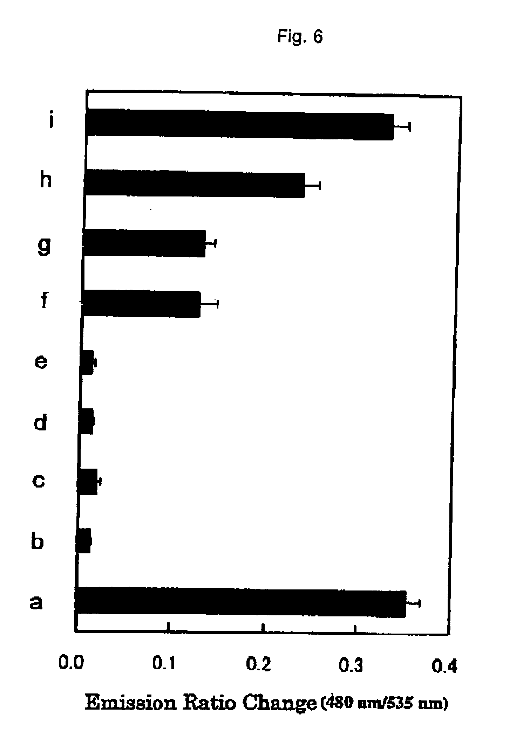

Fig. 6 shows the changes in FRET response, in the Example of this invention, when the probe-expressing CHO-K1 cell is stimulated with E2, ICI 182,780 and OHT (each 1.0 $\mu$M) (a: 1.0 $\mu$M E2; b: 1.0 $\mu$M ICI 182,780; c: 1.0 $\mu$M OHT; d: 1.0 $\mu$M ICI 182,780/1.0 $\mu$M E2; e: 1.0 $\mu$M ICI 182,780/10 $\mu$M E2; f: 1.0 $\mu$M ICI 182.780/100 $\mu$M E2; g: 1.0 $\mu$M OHT/1.0 $\mu$M E2; h: 1.0 $\mu$M OHT/10 $\mu$m E2; i: 1.0 pM OHT/100 $\mu$M E2).

Fig. 7 shows the changes in FRET response when the probe-expressing CHO-K1 cell is stimulated with 10 $\mu$M BADGE.

Fig. 8 shows the changes in FRET response, in the Example of this invention, when the probe-expressing PK-15 cell is stimulated by DHT (1.0 nM, 10 nM, 100 nM) + flutamide or + procymidone (each 10 $\mu$M) or flutamide only (100 nM), procymidone only (100 nM) or DTH only (1.0 nM, 10 nM, 100 nM).

Fig. 9A shows the changes in FRET response when the probe-expressing PK-15 cell is stimulated by 1.0 $\mu$M flutamide; and Fig. 9B shows the changes in FRET response when stimulation is conducted by 100 nM testosterone + 10 $\mu$M flutamide.

Reference letters and numerals in the drawings are as follows.

A: in the coexistence of an agonist
B: in the coexistence of an antagonist
1: a probe for detecting an agonist or an antagonist
2A: a ligand-recognition site (ER $\alpha$-LBD)
2B: a ligand-recognition site (PPAR $\gamma$-LBD)
2C: a ligand-recognition site (AR-LBD)
3: a binding-responsive site (SRC-1 NR box II)
4: a linker
51: a reporter (CFP)
52: a reporter (YFP)
61: an agonist
62: an antagonist
71: a linker
72: a linker

## Best Mode for Carrying Out the Invention

A probe for detecting an agonist or an antagonist of the invention comprises at least four sites each having different function. Thus, as shown in Fig. 1, probe **1** comprises at least the followings:

ligand-recognition site **2** containing a ligand-binding domain of a nuclear receptor (hereinafter referred to as NR-LBD), binding-responsive site **3** containing a peptide chain which specifically binds to coactivator-binding site **22** of NR-LBD, flexible linker **4** which connects ligand-recognition site **2** and binding-responsive site **3**, and two reporters **51** and **52** which generate a detectable signal when they approach by binding of agonist **61** to ligand-recognition site *2*.

Probe *1* shows different conformations in the coexistence of agonist 61 and in the coexistence of antagonist *62,* respectively, and the differences are detectable as a change of signal.

When probe *1* coexists with agonist *61*, agonist *61* recognizes ligand-binding pocket 21 of ligand-recognition site *2* in probe *1* and binds thereto (Fig. 1A-a). A conformation of NR-LBD is then changed and coactivator-binding site *22* is formed in ligand-recognition site *2*. Next, binding-responsive site *3* binds to coactivator-binding site *22* (Fig. 1A-b). As a result, the conformation of probe *1* is changed to approach two reporters *51* and *52*.

On the other hand, when probe *1* coexists with antagonist *62,* antagonist *62* recognizes ligand-binding pocket *21* in ligand-recognition site *2* and binds thereto (Fig. 1B) in the same manner as in the case of agonist *61.* In that case however, no conformational change of NR-LBD occur in the ligand-recognition site and no coactivator-binding site *22* is formed as well. Accordingly, binding of binding-responsive site *3* to ligand-recognition site *2* does not take place and the conformation of probe *1* is not changed either. Thus, two reporters *51* and *52* are left separated.

In such probe *1,* there is no particular limitation for ligand-recognition site *2.* For example, one containing a ligand-binding domain of various nuclear receptors may be used. The nuclear receptors include a glucocorticoid receptor, an estrogen receptor, a progesterone receptor, an androgen receptor, a thyroid gland hormone receptor, a retinoic acid receptor, a vitamin D receptor, a peroxisome growth factor-activating receptor and an orphan receptor to which ligand is unknown. Therefore, in this invention, ligand-recognition site *2* means a ligand-binding domain itself for a nuclear receptor, a partial structure of nuclear receptor containing a ligand-binding domain and a nuclear receptor itself. It goes without saying that the ligand-binding domain of a nuclear receptor is not only known ones but also may be a newly clarified one.

In probe *1* of this invention, binding-responsive site *3* which specifically binds to coactivator-binding site *22* formed by a binding of ligand-recognition site *2* to agonist *61* may be anything including all kinds of synthetic and natural peptides, so far as it contains a peptide chain specifically binding to coactivator-binding site *22* in ligand-recognition site *2*. For example, steroid receptor coactivator 1 (SRC-1) has been known as a coactivator to an estrogen receptor. The SRC-1 contains a nuclear receptor-interacting domain, which consists of equally spaced LXXLL motif (SEQ ID No. 1) or three conservative copies of a leucine-abundant signature motif called NR box. It has been known that such motifs are important for mediating an interaction of SRC-1 with an estrogen receptor (Non-Patent Documents 5 and 23 to 25) and, therefore, when ligand-recognition site *2* is made to contain a ligand-binding domain of an estrogen receptor, a polypeptide having such a motif may be preferably employed as binding-responsive site *3*. Further examples are SRC-1 itself and a coactivator peptide (SEQ ID No. 2) of SRC-1 (Non-Patent Documents 23 to 25).

Such ligand-recognition site *2* and binding-responsive site *3* are linked via flexible linker *4*. Linker *4* may be anything without particular limitation for its structure and sequence so far as it is flexible. Its examples are macromolecular chain and natural or synthetic peptide chain. By means of linker *4*, ligand-recognition site *2* and binding-responsive site *3* are linked each other, as well as they are also able to approach and depart, which also makes it possible for binding-responsive site *3* to approach to or depart from coactivator-binding site *22* in ligand-recognition site *2*.

In probe *1* of this invention, as a site for signaling upon binding of agonist *61,* two reporters *51* and *52* are linked so that their approach is detectable. In that case, the two reporters *51* and *52* should show signal changes with a high precision by responding to the conformational change of probe *1* due to binding of ligand-recognition site *2* to binding-responsive site *3*. Examples of such signal changes are from a renilla luciferase complementation assay (Patent Document 1), a β-galactosidase complementation assay (Non-Patent Document 26) and a monochromatic fluorescence probe (Patent Document 2). In the field of biochemistry, various fluorescent chromophores may be generally used and, a fluorescence resonance energy transfer (hereinafter, referred to as FRET) has been known as quickly responding to a conformational change (Non-Patent Documents 27 to 30).

Accordingly, in probe *1* in this invention, each end of the fusion structure [ligand-recognition site/linker/ binding-responsive site] is linked with reporters *51* and *52* of which approach is detectable. Examples of such reporters are a combination of the N-terminal polypeptide of renilla luciferase with the residual C-terminal polypeptide of the renilla luciferase, a combination of the N-terminal polypeptide of β-galactosidase with the residual C-terminal polypeptide of the β-galactosidase, and a combination of cyan fluorescence protein (CFP), a blue-shifted mutant of green fluorescent protein (GFP) with yellow fluorescent protein (YFP), a red-shifted mutant of GFP.

Particularly, a combination of CFP with YFP is preferred since the CFP acts as a donor for FRET while the YFP acts as an acceptor for FRET whereby they quickly respond to the conformational changes of the probe. In that case, the donor/acceptor may be liked to any of ligand-recognition site *2* binding-responsive site *3*. For example, as shown in Fig. 2A-a and Fig. 2C-a, it is possible to link the CFP to the N-terminal of binding-responsive site *3* and to link the YFP to the C-terminal of the ligand-recognition site. Needless to say, the linking positions of the CFP and the YFP may be reversed.

As mentioned hereinabove, in probe *1* in this invention, each of reporters *51* and *52* may be linked to ligand-recognition site *2* or to binding-responsive site *3* via an appropriate linker *71* or *72* or may be linked directly.

7

The aforementioned probe *1* makes it possible to detect, quantify and screen agonist *61* and antagonist *62.*

As mentioned already, when probe *1* of this invention coexists with an agonist, agonist *61* recognizes ligand-binding pocket *21* of ligand-recognition site *2* and binds thereto. As a result, a conformational change takes place in ligand-recognition site *2* and coactivator-binding site *22* is formed. Then binding-responsive site *3,* which is linked with ligand-recognition site *2* via flexible linker *4,* recognizes coactivator-binding site *22* and binds thereto. As a result, two reporters *51* and *52* approach each other whereby signal changes such as occurrence of luminescence and fluorescence, as well as changes in intensity and wavelength are resulted.

Adding probe *1* to a sample from a patient (such as urine and blood), for example, it is possible to judge as to whether an agonist to a specific nuclear receptor is present in the sample by measuring signal changes and comparing with those of a control (blank). Thus, in case the two reporters *51* and *52* are CFP and YFP for example, a chromophore (supposing *51*) acting as a donor is irradiated and fluorescence intensity of chromophore *51* is measured. If an agonist is present in the sample, the fluorescence intensity ratio of CFP to YFP decreases as compared with that of the blank. On the contrary, if donor chromophore *51* is irradiated and fluorescence intensity of acceptor chromophore *52* is measured in the presence of an antagonist, the fluorescence intensity ratio of CFP to YFP increases as compared with that of the blank. Using a calibration curve between fluorescence intensity and amount (concentration) of agonist, an actual amount of agonist *61* in a sample can be quantified.

The invention of this application also provides a method for screening an agonist using the aforementioned probe *1.* That is, it is possible to screen an agonist by making probe *1* and an agonist candidate substance coexist and measuring signal changes. If the candidate acts as an agonist, two reporters *51* and *52* approach each other according to the aforementioned mechanism and signal changes are observed. On the other hand, if the candidate does not act as an agonist, any signal changes are not observed.

By using probe *1* of this invention, it is also possible to measure the agonistic intensity of the screened candidate. As explained above, a binding of agonist *61* to ligand-recognition site *2* leads to a binding of binding-responsive site *3* to coactivator-binding site *22.* If the binding affinity between agonist *61* and ligand-recognition site *2* is strong, the binding of coactivator-binding site *22* to binding-responsive site *3* is stabilized and the binding of agonist *61* to ligand-recognition site *2* is also stabilized. Accordingly, a dissociation rate of agonist *61* from ligand-recognition site *2* decreases and the reaction equilibrium is shifted to a direction for the formation of an agonist binding. Therefore, in the aforementioned case where two reporters *51* and *52* are CFP and YFP, for example, time-course changes in fluorescence intensity is observed by continuously measuring the fluorescence intensities after addition of agonist *61.* On the other hand, in the case of the binding affinity of agonist *61* to ligand-recognition site *2* being weak, the binding of coactivator-binding site *22* to binding-responsive site *3* is not well stabilized, and hence the binding of agonist *61* to ligand-recognition site *2* is also apt to be dissociated. Thus, reaction equilibrium is maintained or a shift to the direction of dissociation to the agonist is resulted. Accordingly, a time-course shows decrease in the ratio of fluorescence intensities for CFP to YFP after addition of agonist *61* is observed, even if the intensity was temporarily strong.

In the aforementioned method for screening an agonist, it is preferred for maintaining a high precision that there is neither agonist nor antagonist except a candidate substance in the screening.

The invention of this application also provides a method for screening an antagonist using the aforementioned probe *1*. That is, with or without a known agonist such as an endogenous agonist, probe *1* is made to coexist with an excessive amount of an antagonist candidate substance and the signal changes are measured. In the case of only the known agonist and probe *1* being coexisted, two reporters *51* and *52* approach upon binding of the agonist to ligand-recognition site *2* thereby signal is detectable. On the other hand, when an excessive amount of an antagonist candidate substance coexists and exhibits an antagonistic action, it selectively binds to ligand-binding site *22* of ligand-recognition site *2* in probe *1* thereby the signal gradually decreases. In the case where the antagonist candidate substance does not have an antagonistic action, the binding between the agonist and ligand-recognition site *2* of probe *1* is maintained and hence the approach of two reporters *51* and *52* approach is also maintained. Therefore, comparison of signals such as fluorescence intensities with and without antagonist candidate substance makes it possible to precisely judge whether the candidate substance acts as an antagonist.

Concretely, in the case of two reporters *51* and *52* are fluorescence chromophores occurring FRET upon approaching, when donor chromophore (supposing *51*) is irradiated, the fluorescence intensity from chromophore *51* increases with an antagonist, as compared with the case of without antagonist (i.e., with a known agonist only). On the other hand, the donor chromophore (supposing *51*) is irradiated, the fluorescence intensity from acceptor chromophore (supposing *52* in this case) decreases with an antagonist.

In the aforementioned method for screening an agonist or an antagonist, probe *1* could coexist with an agonist, an agonist candidate substance, an antagonist candidate substance in various procedures. For example, probe *1* may coexist with an agonist candidate substance or with a known agonist/excessive amount of antagonist candidate substance in a sample solution. According to such a method, an agonist can be detected, quantified and screened in vitro.

In the screening methods of this application, probe *1* may coexist with an agonist candidate substance or an antagonist candidate substance in cells by introducing an expression vector of probe *1* into cultured cells. As the expression vector, a plasmid vector for animal cells is preferably used. For introducing such a plasmid vector into cells, known methods using electroporation, phosphorylated calcium, liposome and DEAE dextran can employed. Introducing the expression vector of probe *1* into cells and stimulating the cells with an agonist candidate substance, a known agonist, an antagonist candidate substance, an agonist or an antagonist can be screened *in vivo* without destroying the cells.

Further, in accordance with the invention of this application, a transgenic non-human animal having probe *1* in all cells is prepared by introducing a polynucleotide expressing probe *1* into non-human animal totipotent cells and developing the cells into a individual. Preparation of the transgenic non-human animal is in accordance with known methods (such as Non-Patent Document 31). The transgenic non-human animal contains probe *1* in all somatic cells, and therefore probe *1* may coexist with an endogenous agonist in its body. Probe *1* may also coexist with a test substance such as a drug or an endocrine disruptor in the non-human animal by administering the substance into the animal. Measuring the signal changes in cells or tissues, it is possible to screen various substances and further to search influence or effect of a drag, endocrine disruptor or their candidate substance on life activities and diseases. Furthermore, using the transgenic non-human animal, it is possible to visually analyze when and how an inherent agonist is secreted in an animal body.

In the screening method as mentioned hereinabove, an agonist or its candidate substance may be a natural substance or a synthetic substance such as genistein (hereinafter, referred to as Gen; vegetable estrogen), diethylstilbestrol (hereinafter, referred to as DES; stilbene), bisphenol A (hereinafter, referred to as Bis-A; diphenolic substance) and nonylphenol (hereinafter, referred to as NP; alkylphenol) as well as 15-deoxy-$\Delta^{12.14}$-prostaglandin J$_2$ (15d-PGJ$_2$), testosterone, 5$\alpha$-dihydrotestosterone (DHT), etc. With regard to an antagonist or its candidate substance, 4-hydroxytamoxifen (OHT), ICI 182,780, flutamide, cyproterone acetate (CPA), etc. are exemplified without any limitation.

Effects of the inventions are summarized as follows. In accordance with the probe of the first to the sixth inventions, conformation of the nuclear receptor ligand-binding domain is changed upon binding to an agonist thereby a coactivator-binding site is formed. Then, the binding-responsive site linked with the ligand-recognition site via a flexible linker recognizes the coactivator-binding site and binds thereto. As a result, two reporters connected with respective ends of the fusion structure [ligand-recognition site/linker binding-responsive site] approach to emit detectable signals. On the other hand, in such a probe for detecting an agonist or an antagonist, no conformational changes of the nuclear receptor ligand-binding domain occur upon binding an antagonist, and hence the coactivator-binding site is not formed. Therefore, the two reporters linked to the both ends of the fusion structure are still separated and no detectable signal is generated. Accordingly, an agonist and an antagonist to the nuclear receptor can be selectively detected and quantified by using the probe. It is also possible to judge quickly and precisely whether a substance may act as an agonist or an antagonist to the nuclear receptor.

In the seventh invention, the probe coexists with an agonist candidate substance and signal changes with and without the candidate are measured. It is able to screen whether the candidate substance acts as an agonist to the nuclear receptor easily and highly precisely.

In the eighth invention, the probe coexists with an agonist candidate substance in cells by introducing a polynucleotide expressing the probe into the cells. It is possible to judge whether the candidate substance acts as an agonist *in vivo*.

In the ninth invention, the probe coexists with an agonist candidate substance in all cells of non-human animal or its progeny (13th invention) by introducing a polynucleotide expressing the probe into totipotent cells and developing the cells into individual. It is possible to monitor an influence or effect of the substance to life activities and diseases of the non-human animal. It is further possible to visually analyze when and how an inherent agonist (such as estrogen) is secreted using the non-human animal.

In the tenth invention, the probe and an excessive amount of an antagonist candidate substance coexist with a known agonist, and signal changes with and without the antagonist candidate substance are measured. It is possible to judge whether the candidate substance acts as an antagonist to the nuclear receptor easily and highly precisely.

In the eleventh invention, the probe coexists with a known agonist and an antagonist candidate substance in cells by introducing a polynucleotide expressing the probe into the cells. It is possible to judge whether the candidate substance acts as an antagonist *in vivo*.

In the twelfth invention, the probe coexists with a known agonist and an antagonist candidate substance in all cells of non-human animal or its progeny (13th invention) by introducing a polynucleotide expressing the probe into totipotent cells and developing the cells into individual. it is possible not only to judge whether the candidate substance acts as an antagonist to the nuclear receptor, but also to monitor an influence or effect of the candidate substance to life activities and diseases of the non-human animal.

As hereunder, embodiments of this invention will be further illustrated with Examples. Needless to say, this invention is not limited to the following Examples but various modifications are possible in particulars.

**Examples**

**[Preparation]**

(1) Materials

Ham's F-12 medium, fetal calf serum (FCS), Hanks' balanced salt solution (HBSS) and lipofectAMINE 2000 reagent were purchased from Life Technologies (Rockville, M.D). Dulbecco-modified Eagle's medium (DMEM), trypsin-EDTA, E2, DES, OHT and Gen were purchased from Sigma Chemicals Co. (St. Louis, MO). Anti-GFP antibody was obtained from Clontech, (Palo Alto, CA). All cloning enzymes were from Takara Biomedical (Tokyo, Japan). The hER α cDNA plasmid was purchased from American Type Culture Collection (ATCC, VA, USA). A mammalian expression vector pcDNA3.1 (+) was from Invitrogen Co. (Carlbad, CA). Bis-A. NP and ICI 182,780 were from Wako Pure Chemicals Industries, Ltd. (Osaka, Japan). All other chemicals used were of analytical reagent grade.

(2) Plasmid Constructions

(A) Probe for detecting an agonist or an antagonist to an estrogen receptor

To prepare the cDNAs for the constructs shown in Fig. 2A, fragments of ECFP (SEQ ID No: 3) (1-238aa), EYFP (SEQ ID No: 4) (1-238aa), human estrogen receptor αLBD (SEQ ID No: 5) (305-550aa) (hereinafter, referred to as ER α-LBD), flexible linker (GGNGG) (SEQ ID No: 6) and steroid receptor coactivator 1 NR box II (SEQ ID No: 2) (687-697aa) were generated by standard polymerase chain reaction (PCR) to attack a Kozak sequence (shown as Kz in the drawing) and restriction sites shown in constructs.

From the constructs, a probe having CFP (donor) and YFP (acceptor) on both ends of a fusion protein is prepared, in which the fusion protein consists of a coactivator peptide (SEQ ID No: 2) (Non-Patent Documents 23 to 25) of a steroid receptor coactivator 1 (SRC-1) and ER αLBD linked via a short flexible linker (SEQ ID No: 3).

All PCR fragments were sequenced with an ABI310 genetic analyzer. This cDNA was inserted at HindIII and XhoI sites of mammalian expression vector pcDNA3.1(+).

(B) Probe for detecting an agonist or an antagonist to a peroxisome growth factor-activating receptor

According to nearly the same procedure as in the case of the above (A), a plasmid was constructed. To prepare the cDNAs for the constructs shown in Fig. 2B, fragments of ECFP (SEQ ID No: 3) (1-238aa), EYFP (SEQ ID No: 4) (1-238aa), peroxisome growth factor-activating receptor γLBD (SEQ ID No: 8) (235-505aa) (hereinafter, referred to as PPAR γ-LBD), flexible linker (GGNGG)$_6$ (SEQ ID No: 9) and steroid coactivator 1NR box II (SEQ ID No: 2) (687-697aa) were generated by standard polymerase chain reaction (PCR) to attack a Kozak sequence (shown as Kz in the drawing) and restriction sites shown in constructs.

From the constructs, a probe having CFP (donor) and YFP (acceptor) on both ends of a fusion protein is prepared, in which the fusion protein consists of a coactivator peptide (SEQ ID No: 2) (Non-Patent Documents 23 to 25) of a steroid receptor coactivator 1 (SRC-1) and PPAR γ-LBD kinked via a short flexible linker (SEQ ID No: 3).

All PCR fragments were sequenced with an ABI310 genetic analyzer. This cDNA was inserted at HindIII and XhoI sites of mammalian expression vector pcDNA3.1(+).

(C) Probe for detecting an agonist or an antagonist to androgen

According to nearly the same procedure as in the cases of the above (A) and (B), a plasmid was constructed. To prepare the cDNAs for the constructs shown in Fig. 2C, fragments of ECFP (SEQ ID No: 3) (1-238aa), EYFP (SEQ ID No: 4) (1-238aa), human androgen receptor LBD (SEQ ID No: 10) (672-910aa) (hereinafter, referred to as AR-LBD), flexible linker (GGNGG)$_3$ (SEQ ID No: 11) and steroid coactivator 1NR box II (SEQ ID No: 2) (687-697aa) were generated by standard polymerase chain reaction (PCR) to attack a Kozak sequence (shown as Kz in the drawing) and restriction sites shown in constructs.

From the constructs, a probe having CFP (donor) and YFP (acceptor) on both ends of a fusion protein is prepared, in which the fusion protein consists of a coactivator peptide (SEQ ID No: 2) (Non-Patent Documents 23 to 25) of a steroid receptor coactivator 1 (SRC-1) and AR-LBD kinked via a short flexible linker (SEQ ID No: 3).

All PCR fragments were sequenced with an ABI310 genetic analyzer. This cDNA was inserted at HindIII and XhoI sites of mammalian expression vector pcDNA3.1(+).

(3) Cell Culture and Transfection

Each cell was cultured in Ham's F-12 medium supplemented with 10% fetal calf serum (FCS)b and Dulbecco's modified eagle medium supplemented with 10% FCS, 1.0 mM sodium pyruvate and 0.1 mM nonessential amino acids respectively at 37°C in a humidified atmosphere 5% $CO_2$. Cells were trasnfected with an expression vector pcDNA3.1 (+) containing a probe in the presence of lipofectAMINE 2000 reagent in glass bottom dishes.

The cells were Chinese hamster ovary (CHO-K1) cells for a probe for detecting an agonist or an antagonist to an estrogen receptor or a peroxisome growth factor-activating receptor, and pig kidney (PK-15) cells for a probe for detecting an agonist or an antagonist to an androgen receptor.

In 12 to 24 after transfection, cells were ready for imaging.

(4) Immunoblot Analysis for Protein Expression

The cell lysate of CHO-K1 or PK-15 transfected with expression vector pcDNA3.1 (+) encoding each of the probes shown in Fig. 2A to Fig. 2C was subjected to SDS-PAGE using 10% polyacrylamide gel electrophoresis and electrophoretically transferred onto a nitrocellulose membrane.

The membrane was probed with anti-GFP antibody (1:500 in 1% skim milk in TBST (50 mM Tris-HCl pH 8.0, 150 mM NaCl. 0.05% Tween 20)) and then with alkaline phosphatase-labeled anti-rabbit antibody (1:5000 in 1 % skim milk in TBST).

The protein expression was analyzed by an image analyzer (LAS-1000 plus, Fuji Photo Film Co., Tokyo, Japan).

(5) Imaging of Cells

Before imageing, the culture medium was replaced with Hanks' balanced salt solution. Within 12-24 hours after the transfusion, the cells were imaged at room temperature on a Carl Zeiss Axiovert 135 microscope with a cooled charge-coupled device camera MicroMAX (Roper Scientific Inc., Tucson, AZ), controlled by MetaFluor (Universal Imaging, West Chester, PA).

The exposure time at 440 $\pm$ 10 nm excitation was 100 ms. The fluorescent images were obtained through 480 $\pm$ 15 nm and 535 $\pm$ 12.5 nm filters with a 40x oil immersion objective (Carl Zeiss, Jena, Germany).

**Example 1**

**Evaluation of responding property of probe for detecting an agonist or an antagonist**

(1) Probe for detecting an agonist or an antagonist to an estrogen receptor

(1-1) 17β-Estradiol (E2) having the strongest activity among esterogens was used as an agonist and a response of the probe was evaluated.

CHO-K1 cells expressing the probe (Fig. 2A-a) were stimulated by 100 nM E2 and the event was observed following the time course of the changes in FRET. The cell images were recorded before and at different intervals after E2 stimulation as shown Fig. 3A, where the 480/535 nm emission ratio was represented by pseudocolor images.

As shown in Fig. 3A, upon stimulation with 100 nM E2, a blue shift of the pseudocolor was observed as a decrease in the CFP/YFP emission ratio (increase in FRET).

The decrease in emission ratio was observed for a few seconds and reached a plateau within 1000 seconds (Fig. 4A-a). On the other hand, no detectable change was noted with the blank experiment (in the absent of E2) under otherwise identical conditions (Fig. 4A-b).

(1-2) To confirm that the increase in the FRET was triggered by the ligand-recognition site (ER αLBD)/the binding-responsive site (LXXLL motif) interaction upon E2 stimulation, a mutant probe (SEQ ID No: 7) was prepared by replacing all the hydrophobic leucine (L) residues of LXXLL motif with alanine (A) residues (Fig. 2A-b).

As shown in Fig. 4c, the CHO-K1 cell expressing the mutant probe did not show any significant change in the emission ratio of CFP/YFP upon E2 stimulation.

Taken all together, it was concluded that the FRET response was due to the recruitment of the binding-responsive site (LXXLL motif) to the ligand-recognition site (ER αLBD), which was agonist dependent.

As reported previously (Non-Patent Documents 5 and 23 to 25), the hydrophobic leucine residues of LXXLL motif were confirmed to play a significant role for the interaction with a coactivator-binding site of ER α-LBD.

(1-3) According to the X-ray crystallographic analysis of the E2/ER $\alpha$LBD complex, E2 is completely enveloped with the hydrophobic ligand binding pocket (450Å$^3$: about twice the molecular volume of E2, 245Å$^3$) in the ER $\alpha$LBD in such a way to adopt low energy conformation (Non-Patent Document 32).

The Helix 12 is positioned over the ligand binding pocket, thereby forming a hydrophobic groove for recruitment and interaction of the hydrophobic LXXLL motif, which is stabilized by forming van der Waals contacts between side chains of three leucine residues of LXXLL motif with the side chains of Met 543, Lys 642 and Glu 542 residues of ER $\alpha$LBD (Non-Patent Document 33).

From these results, it was confirmed that CFP and YFP approached by an interaction of the ligand-recognition site (ER $\alpha$LBD) and the binding-responsive site (LXXLL motif) and accordingly FRET was resulted.

(2) Probe for detecting an agonist or an antagonist to a peroxisome proliferator-activated receptor

(2-1) Response of the probe was evaluated by using an intrinsic agonist, 15-deoxy-$\Delta^{12,14}$-prostaglandin J$_2$ (15d-PGJ$_2$). Response of the probe to pioglitazone, an anti-diabetic agent, was also evaluated.

CHO-K1 cells expressing the probe (Fig. 2B) were stimulated by 10 $\mu$M 15d-PGJ$_2$ and the event was observed by following time course fo the changes in FRET.

The decrease in CFP/YFP emission ratio (480/535 nm) (increase in FRET) was observed for 400 seconds and reached a plateau within 1000 seconds (Fig. 4B-a). Fig. 4B-b shows a relation (reaction curve) of the concentration of 15d-PGJ$_2$ with the changes in emission ratio.

(2-2) Time course changes in FRET were also observed after stimulating CHO-K1 cells expressing the probe (Fig. 2B) with 1.0 $\mu$M pioglitazone.

The decrease in CFP/YFP emission ratio (480/535 nm) (increase in FRET) was observed for 100 seconds and reached a plateau within 100 seconds (Fig. 4C-a). Fig. 4C-b shows a relation (reaction curve) of the concentration of pioglitazone with the changes in emission ratio.

(3) Probe for detecting an agonist or an antagonist to an androgen receptor

(3-1) Response of the probe was evaluated by using an agonist, 5$\alpha$-Dihydrotestosterone (DHT).

PK-15 cells expressing the probe (Fig. 2C-a) were stimulated by 100 nM DHT and the event was observed by following time course of the changes in FRET. The cell images were recorded before and at different time intervals after DHT stimulation as shown in Fig. 3B, were the 480/535 nm emission ratio was represented by pseudocolor images.

Upon stimulation with 100 nM DHT, a blue shift of the pseudocolor was observed as a decrease in the CFP/YFP emission ratio (increase in FRET).

The decrease in emission ratio was observed for a few seconds and reached a plateau within 1200 seconds (Fig. 4D-a). On the other hand, no detectable change was noted with the blank experiment (in the absent of DHT) under otherwise identical conditions (Fig. 4D-b).

(3-2) To confirm that the increase in the FRET was triggered by the ligand-recognition site (AR- LBD)/the binding-responsive site (LXXLL motif) interaction upon DHT stimulation, a mutant probe (SEQ ID No: 7) was made by replacing all the hydrophobic leucine (L) residues of LXXLL motif with alanine (A) residues (Fig. 2B-b).

As shown in Fig. 4c, in the CHO-K1 cells expressing the mutant probe did not show any significant change in the emission ratio of CFP/YFP upon DHT stimulation.

Taken all together, it was concluded that the FRET response was due to the recruitment of the binding-responsive site (LXXLL motif) to the ligand-recognition site (AR- LBD), which was agonist dependent. This conclusion was similar to that for the probe for detecting an agonist or an antagonist to an estrogen receptor.

**Example 2**

**Screening of an extrinsic agonist using a probe for detecting an agonist or an antagonist**

(1) Probe for detecting an agonist or an antagonist to an estrogen receptor

Extrinsic estrogens such as DES, Gen, Bis-A and NP were assessed for their abilities to confer estrogenic activity by using CHO-K1 cells expressing the probe.

These compounds were tested over concentration range from $1.0 \times 10^{-4}$ to $1.0 \times 10^{-11}$ M.

Fig. 5A shows relations between concentrations of each compound and changes in emission ratio (dose-response curves).

$EC_{50}$ values (the effective concentration of a ligand to induce a 50% response as a result of the binding-responding site (LXXLL motif) recruitment to the ligand-recognition site (ER $\alpha$LBD) values were determined from the response curves on Fig. 5A, which were $0.8 \times 10^{-8}$ M, $1.3 \times 10^{-8}$ M, $6.5 \times 10^{-8}$ M, $0.26 \times 10^{-6}$ M and $0.42 \times 10^{-6}$ M for E2, DES, Gen, NP and Bis-A, respectively. The $ED_{50}$ values were converted to relative recruitment ability (RRA; Non-Patent Document 34) to compare the relative abilities of the tested compounds to promote the recruitment of the binding-responsive site to the ligand-recognition site.The RRA were calculated using the following equation.

$$RRA = (\text{concentration E2 [50\% response]}/$$

$$\text{concentration EDC [50\% response]}) \times 100$$

The RRA value for E2 was arbitrarily set at 100. The RRA values for E2, DES, Gen, NP and Bis-A were thereby obtained as 100, 60, 12, 3.0 and 1.9, respectively.

Thus, the ability of the tested compounds to induce the binding-responsive site to the ligand-recognition site was as follows in the decreasing order:

$$E2 > DES > Gen > NP > Bis-A.$$

From the above results, it was confirmed that the subtle difference in FRET response level reflects the potency of the compounds to induce a ligand specific conformational change in ER $\alpha$LBD to recruit the LXXLL motif within the probe.

The primary purpose of E2 binding to ER is to induce a conformational change in the tertiary structure of ER, such that the LBD is in a position to mediate the assembly of the basal transcription machinery following the recruitment of coactivator (Non-Patent Documents 4 and 5).

However, the actual conformational changes, which is the repositioning of the helix 12, in the tertiary structure of ER induced by extrinsic estrogens may differ from that of E2 (Non-Patent Documents 24, 25, 35 and 36), due to difference in the steric and electrostatic properties of various ligands.

In this regard, Routleddge et al. determined the ability of each compound for promoting recruitment of ER to coactivator protein using a GST pull-down assay and obtained the same results (Non-Patent Document 34).

(2) Probe for detecting an agonist or an antagonist to an androgen receptor

Androgen agonist DHT, as well as testosterone, progesterone and cortisol that are androgen antagonists were assessed for their abilities to confer andogenic activity by using PK-15 cells expressing the probe.

These compounds were tested over concentration range from $1.0 \times 10^{-4}$ to $1.0 \times 10^{-11}$ M.

Fig. 5B shows dose-dependent curves between concentrations of each compound and changes in emission ratios.

From Fig. 5B. $EC_{50}$ values (concentration of a compound necessary for occurrence of 50% of the reaction between the ligand-recognition site (AR-LBD) and the binding-responding site (LXXLL motif)) for DHT, testosterone and progesterone were $1.1 \times 10^{-9}$ M, $1.7 \times 10^{-8}$ M and $4.7 \times 10^{-7}$ M respectively. The cortisol did not show the response at any tested concentration.

The FRET response of DHT was the same to that of 10-fold higher concentration of testosterone.

In contrast, the progesterone did not display the same magnitude of the FRET level as induced by the DHT and the testosterone even with its highest concentration used in this experiment.

From the above results, it was confirmed that the difference in FRET response level reflects the potency of the compounds to induce a ligand specific conformational change in AR-LBD to recruit the coactivator peptide within the probe.

**Example 3**

**Discrimination of agonist from antagonist**

(1) Probe for detecting an agonist or an antagonist to an estrogen receptor

(1-1) CHO-K1 cells expressing the probe were stimulated by 1.0 $\mu$M E2, ICI 182,780 and OHT each, and changes in their FRET responses were monitored. The ICI 182,780 and OHT are antagonists to an estrogen receptor.

A high increase in the FRET was observed in the case of E2 (Fig. 6-a), while no increase in the FRET was observed in the case of ICI 182,780 and OHT (Figs. 6-b and 6-c).

(1-2) The ability of E2 to displace the ICI 182, 780 and OHT from the ligand-recognition domain was determined.

A 1.0 $\mu$M concentration of ICI 182,780 was added to three different glass-bottom dishes containing CHO-K1 cells expressing the probe and the resultant mixture was incubated for 15 minutes at room temperature. One, 10, and 100 $\mu$M E2 were added to first. second and third dishes, respectively, without washing the ICI 182,780, and the changes were monitored in the FRET response.

No change in the FRET level was observed upon adding 1.0 $\mu$M and 10 $\mu$M E2 in the presence of 1.0 $\mu$M of ICI 182,780 (Figs. 6-d, -e), while 100 $\mu$M E2 induced a changes in the FRET level, which was ~40% of that induced by 1.0 $\mu$M E2 alone (Fig. 6-f).

Similarly, addition of 1.0. 10, and 100 $\mu$M E2 in the presence of 1.0 $\mu$M OHT exhibited increases in the FRET responses by 40, 65, and 90%, respectively, that with 1.0 $\mu$M E2 alone (Figs. 6-g, -h, -i).

The results demonstrates that E2 (agonist) promotes interaction between the ligand-recognition site (ER $\alpha$LBD) and the binding-responding site (LXXLL motif) of the probe in living cells, whereas antagonists, ICI 182, 780 and OHT, block this interaction in a manner that was not completely recovered even by using a 100-fold higher concentration of agonist.

Accordingly, it was confirmed that the signal from the probe in the presence of an agonist was blocked by coexistence with an antagonist.

(1-3) These results can be discussed in the light of X-ray crystallographic studies of the ER $\alpha$LBD in the presence of an agonist and an antagonist (Non-Patent Documents 32 and 33).

The agonist binding induces a conformational change in the ligand-binding domain, exposing a coactivator-binding site on the surface of the ligand-binding domain due to the alignment of helix 12 over the ligand binding cavity. On the other hand, the antagonists are able to prevent the proper alignment of helix12 through direct steric effects between their characteristic basic bulky side chain and helix12. Consequently, the coactivator-binding site is not properly formed and the ligand-binding domain (ER $\alpha$-LBD in the Examples) is unable to interact with the nuclear receptor-interacting domain of the coactivator (LXXLL motif in the Examples).

Therefore, the transfer of energy from CFP to YFP in the probe was possible in the case of agonist (E2), but blocked in the case of antagonist (ICI 182,780 and OHT).

Thus, the binding-responsive site recruitment ability of the ligand-recognition site in response to different candidates enabled us to discriminate agonists from antagonists.

(2) Probe for detecting an agonist or an antagonist to a peroxisome proliferator-activated receptor

CHO-K1 cells expressing the probe were stimulated by 10 $\mu$M bisphenol A diglycidyl ether (BADGE) which was an antagonist to a peroxisome proliferator-activated receptor and the changes in the FRET responses were monitored.

As shown in Fig. 7, there was no significant change in the emission ratio even when BADGE was added.

(3) Probe for detecting an agonist or an antagonist to an androgen receptor

(3-1) PK-15 cells expressing the probe were stimulated by DHT (1.0 nM, 10 nM and 100 nM) and flutamide and procymidone (each 10 $\mu$M) which are antagonists to an estrogen receptor and changes in the FRET response were monitored.

As shown in Fig. 8, addition of DHT significantly increases the FRET in a dose dependent manner, while addition of flutamide or procymidone (each 100 nM) produced no significant increase in the FRET.
In the cases of DHT + flutamide or DHT + procymidone, the FRET level increased in a dose dependent manner of DHT.

(3-2) PK-15 expressing the probe were stimulated by flutamide (1.0 $\mu$M) alone or by flutamide (10 $\mu$M) + testosterone (100 nM), and the changes in the FRET response was monitored. In the case of flutamide (10 $\mu$M) + testosterone (100 nM), testosterone was firstly added and then flutamide was added at the timing of before or after reaching a plateau.

As shown in Fig. 9A, no change was particularly observed with stimulation of flutamide (1.0 $\mu$M) alone. On the other hand, in the case of stimulation by flutamide (10 $\mu$M) + testosterone (100 nM), the emission ratio decreased immediately after addition of testosterone and reached a plateau within about 3000 seconds. However, upon stimulation of flutamide, the emission ratio tended to increase.

**Industrial Applicability**

As fully illustrated hereinabove, a probe for selectively detecting and quantifing an agonist or an antagonist to a nuclear receptor is provided. Using the probe, it is possible to quickly and precisely judge whether a specific substance acts as an agonist or as an antagonist to the nuclear receptor. Accordingly, a highly efficient screening for endocrine disruptors or drugs for specific diseases could be provided.

**Claims**

1. A probe for detecting an agonist (61) or an antagonist (62) to a nuclear receptor, in which, at least, a ligand-recognition site (2) containing a ligand-binding domain of the nuclear receptor is connected with a binding-responsive site (3) containing a peptide chain that specifically binds to a coactivator-binding site (22) in the ligand-binding domain by a flexible linker (4) to construct a fusion structure [ligand-recognition site/linker/binding-responsive site], and two reporters (51 and 52) are connected with the respective ends of the fusion structure.

2. The probe of claim 1, wherein the ligand-recognition site (2) contains a ligand-binding domain of a nuclear receptor selected from the group including glucocorticoid receptor, estrogen receptor, progesterone receptor, peroxisome proliferator-activated receptor, androgen receptor, thyroid gland hormone receptor, retinoic acid receptor, vitamin D receptor and orphan receptors.

3. The probe of claim 1, wherein the ligand-recognition site (2) is an estrogen receptor $\alpha$ ligand-binding domain, a peroxisome growth factor activation receptor ligand-binding domain or an androgen receptor ligand-binding domain.

4. The probe of claim 3, wherein the binding-responsive site (3) is a nuclear receptor interaction domain peptide of steroid receptor coactivator 1.

5. The probe of claim 3, wherein the binding-responsive site (3) contains the motif of SEQ ID No: 1.

**6.** The probe of any of claims 1 to 5, wherein the two reporters (51 and 52) are a yellow fluorescent protein and a cyan fluorescent protein.

**7.** A method for screening an agonist to nuclear receptor, which comprises making any of the probes of claims 1 to 6 coexist with an agonist candidate substance, and measuring changes in signals with and without the agonist candidate substance.

**8.** The method for screening an agonist according to claim 7, wherein the probe coexists with the agonist candidate substance in cells by introducing a polynucleotide expressing the probe into the cells.

**9.** The method for screening an agonist according to claim 7, wherein the probe coexists with the agonist candidate substance in all cells of a non-human animal or its progeny by introducing a polynucleotide expressing the probe into a non-human animal totipotent cell and developing the cell into a individual animal.

**10.** A method for screening an antagonist to nuclear receptor, which comprises making any of the probes of claims 1 to 6 coexist with an excessive amount of antagonist candidate substance and a known agonist, and measuring changes in a signal with and without the antagonist candidate substance.

**11.** The method for screening an antagonist according to claim 10, wherein the probe coexists with the agonist and the antagonist candidate substance in cells by introducing a polynucleotide expressing the probe into the cells.

**12.** The method for detecting an antagonist according to claim 10, wherein the probe coexists with the agonist and the antagonist candidate substance in all cells of a non-human animal or its progeny by introducing a polynucleotide expressing the probe into a non-human animal totipotent cell and developing the cell into an individual animal.

**13.** A non-human animal or its progeny, which is established by introducing a polynucleotide expressing any of the probes of claims 1 to 6 into non-human animal totipotent cell and developing the cell into an individual animal.

**Patentansprüche**

**1.** Sonde zum Nachweis eines Agonisten (61) oder eines Antagonisten (62) an einem nukleären Rezeptor, in welcher mindestens eine Ligandenerkennungsstelle (2), die eine Ligandenbindungsdomäne des nukleären Rezeptors enthält, mit einer bindungsresponsiven Stelle (3) verbunden ist, die eine Peptidkette enthält, welche sich spezifisch über einen flexiblen Linker (4) an eine Coaktivator-Bindungsstelle (22) in der Ligandenbindungsdomäne bindet, um eine Fusionsstruktur [Ligandenerkennungsstelle/Linker/bindungsresponsive Stelle] zu bilden, und in welcher zwei Reporter (51 und 52) mit den jeweiligen Enden der Fusionsstruktur verbunden sind.

**2.** Sonde nach Anspruch 1, wobei die Ligandenerkennungsstelle (2) eine Ligandenbindungsdomäne eines nukleären Rezeptors enthält, ausgewählt aus der Gruppe, die Glukokortikoidrezeptor, Östrogenrezeptor, Progesteronrezeptor, Peroxisom-Proliferator-aktivierter-Rezeptor, Androgenrezeptor, Thyroxin-Rezeptor, Retinsäure-Rezeptor, Vitamin-D-Rezeptor und Orphan-Rezeptoren umfasst.

**3.** Sonde nach Anspruch 1, wobei die Ligandenerkennungsstelle (2) eine Ligandenbindungsdomäne des Östrogenrezeptors $\alpha$, eine Ligandenbindungsdomäne des Peroxisom-Wachstumsfaktor-aktivierten-Rezeptors oder eine Ligandenbindungsdomäne des Androgenrezeptors ist.

**4.** Sonde nach Anspruch 3, wobei die bindungsresponsive Stelle (3) ein nukleäres Rezeptorinteraktionsdomäne-Peptid oder ein Steroidrezeptor-Coaktivator 1 ist.

**5.** Sonde nach Anspruch 3, wobei die bindungsresponsive Stelle (3) das Motiv von SEQ ID NR:1 enthält.

**6.** Sonde nach einem der Ansprüche 1 bis 5, wobei die beiden Reporter (51 und 52) ein gelb fluoreszierende Protein und ein cyan fluoreszierendes Protein sind.

**7.** Verfahren zum Screenen eines Agonisten an einem nukleären Rezeptor, welches das Veranlassen, dass die Sonden der Ansprüche 1 bis 6 zusammen mit einer Agonist-Kandidatensubstanz vorliegen und die Messung von Veränderungen in Signalen mit und ohne die Agonist-Kandidatensubstanz umfasst.

8. Verfahren zum Screenen eines Agonisten nach Anspruch 7, wobei die Sonde zusammen mit der Agonist-Kandidatensubstanz in Zellen vorliegt, indem ein Polynukleotid, das die Sonde in den Zellen exprimiert, eingeschleust wird.

9. Verfahren zum Screenen eines Agonisten nach Anspruch 7, wobei die Sonde zusammen mit der Agonist-Kandidatensubstanz in allen Zellen eines nichthumanen Tieres oder seiner Nachkommenschaft vorliegt, indem ein Polynukleotid, das die Sonde in einer totipotenten Zelle eines nichthumanen Tieres exprimiert, eingeschleust wird, und die Zelle zu einem Einzeltier entwickelt wird.

10. Verfahren zum Screenen eines Antagonisten an einem nukleären Rezeptor, welches das Veranlassen, dass die Sonden nach Ansprüchen 1 bis 6 zusammen mit einer überschüssigen Menge einer Antagonist-Kandidatensubstanz und einem bekannten Agonisten vorliegen, und die Messung von Veränderungen in einem Signal mit und ohne die Antagonist-Kandidatensubstanz umfasst.

11. Verfahren zum Screenen eines Antagonisten nach Anspruch 10, wobei die Sonde zusammen mit dem Agonisten und der Antagonist-Kandidatensubstanz in Zellen vorliegt, indem ein Polynukleotid, das die Sonde in den Zellen exprimiert, eingeschleust wird.

12. Verfahren zum Nachweis eines Antagonisten nach Anspruch 10, wobei die Sonde zusammen mit dem Agonisten und der Antagonist-Kandidatensubstanz in allen Zellen eines nichthumanen Tieres oder seiner Nachkommenschaft vorliegt, indem ein Polynukleotid, das die Sonde in einer totipotenten Zelle eines nichthumanen Tieres exprimiert, eingeschleust wird und die Zelle zu einem Einzeltier entwickelt wird.

13. Nichthumanes Tier oder seine Nachkommenschaft, welche(s) entsteht, indem ein Polynukleotid, das eine der Sonden der Ansprüche 1 bis 6 in einer totipotenten Zelle eines nichthumanen Tieres exprimiert, eingeschleust wird und die Zelle zu einem Einzeltier entwickelt wird.

**Revendications**

1. Sonde de détection d'un agoniste (61) ou d'un antagoniste (62) d'un récepteur nucléaire, dans laquelle, au moins, un site de reconnaissance du ligand (2) contenant un domaine de liaison du ligand du récepteur nucléaire est connecté à un site sensible à la liaison (3) contenant une chaîne peptidique qui se lie spécifiquement à un site de liaison du coactivateur (22) dans le domaine de liaison du ligand par un lieur flexible (4) pour construire une structure de fusion [site de reconnaissance du ligand/lieur/site sensible à la liaison], et deux rapporteurs (51 et 52) sont connectés aux extrémités respectives de la structure de fusion.

2. Sonde selon la revendication 1, dans laquelle le site de reconnaissance du ligand (2) contient un domaine de liaison du ligand d'un récepteur nucléaire choisi dans le groupe constitué par le récepteur des glucocorticoïdes, le récepteur des oestrogènes, le récepteur de la progestérone, le récepteur activé par les proliférateurs de peroxysomes, le récepteur d'androgène, le récepteur des hormones de la glande thyroïde, le récepteur de l'acide rétinoïque, le récepteur de la vitamine D et les récepteurs orphelins.

3. Sonde selon la revendication 1, dans laquelle le site de reconnaissance du ligand (2) est un domaine de liaison du ligand du récepteur des oestrogènes a, un domaine de liaison du ligand du récepteur activé par les proliférateurs de peroxysomes ou un domaine de liaison du ligand du récepteur d'androgène.

4. Sonde selon la revendication 3, dans laquelle le site sensible à la liaison (3) est un peptide du domaine d'interaction du récepteur nucléaire du coactivateur du récepteur stéroïdien 1.

5. Sonde selon la revendication 3, dans laquelle le site sensible à la liaison (3) contient le motif de SEQ ID n° : 1.

6. Sonde selon l'une quelconque des revendications 1 à 5, dans laquelle les deux rapporteurs (51 et 52) sont une protéine fluorescente jaune et une protéine fluorescente cyan.

7. Procédé de détection d'un agoniste du récepteur nucléaire, qui comprend l'étape consistant à faire coexister l'une quelconque des sondes selon les revendications 1 à 6 avec une substance candidate agoniste, et à mesurer des variations de signaux avec et sans la substance candidate agoniste.

8. Procédé de détection d'un agoniste selon la revendication 7, dans lequel la sonde coexiste avec la substance candidate agoniste dans des cellules en introduisant un polynucléotide exprimant la sonde dans les cellules.

9. Procédé de détection d'un agoniste selon la revendication 7, dans lequel la sonde coexiste avec la substance candidate agoniste dans toutes les cellules d'un animal non humain ou sa progéniture en introduisant un polynucléotide exprimant la sonde dans une cellule totipotente d'animal non humain et en développant la cellule dans un animal distinct.

10. Procédé de détection d'un antagoniste du récepteur nucléaire, qui comprend l'étape consistant à faire coexister l'une quelconque des sondes selon les revendications 1 à 6 avec une quantité excessive de substance candidate antagoniste et d'un agoniste connu, et à mesurer des variations de signaux avec et sans la substance candidate antagoniste.

11. Procédé de détection d'un antagoniste selon la revendication 10, dans lequel la sonde coexiste avec l'agoniste et la substance candidate antagoniste dans des cellules en introduisant un polynucléotide exprimant la sonde dans les cellules.

12. Procédé de détection d'un antagoniste selon la revendication 10, dans lequel la sonde coexiste avec l'agoniste et la substance candidate antagoniste dans toutes les cellules d'un animal non humain ou sa progéniture en introduisant un polynucléotide exprimant la sonde dans une cellule totipotente d'animal non humain et en développant la cellule dans un animal distinct.

13. Animal non humain ou sa progéniture, qui est établi en introduisant un polynucléotide exprimant l'une quelconque des sondes selon les revendications 1 à 6 dans une cellule totipotente d'animal non humain et en développant la cellule dans un animal distinct.

Fig. 1

EP 1 752 544 B1

a

Kz | HindIII | 51 | EcoR1 | 3 | 4 | Kpn1 | 2 | BamH1 | 52 | Xho1

CFP | LXXLL | GGNGG | ERα LBD | YFP

b

Kz | HindIII | EcoR1 | Kpn1 | BamH1 | Xho1

CFP | AXXAA | GGNGG | ERα LBD | YFP

51 | 3 | 4 | 2 | 52

Fig. 2

Fig. 3

Fig. 4

Fig. 5

Fig. 6

Emission Ratio Change (480 nm/535 nm)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003145466 A
- JP 2003301259 A
- JP 2004035678 A

### Non-patent literature cited in the description

- LLOPIS J. et al. *PNAS,* 2000, vol. 97 (8), 4363-4368
- CIOCCA, D. R. ; ROIG, L. M. V. *Endocr. Rev.,* 1995, vol. 16, 35-62
- TSAI, M. J. ; O'MALLEY, B. W. *Annu. Rev. Biochem.,* 1994, vol. 63, 451-486
- NILSSON, S. et al. *Physiological Rev.,* 2001, vol. 81, 1535-1565
- MCKENNA, N. J. et al. *Endocr. Rev.,* 1999, vol. 20, 321-344
- GEE, A. C. et al. *Mol. Endocrinol.,* 1999, vol. 13, 1912-1923
- KORACH, K. S. *Endocrinology,* 1993, vol. 132, 2277-2278
- SHELBY, M. D. et al. *Environ. Health Perspect.,* 1996, vol. 104, 1296-1300
- OOSTERKAMP, A. J. et al. *Anal. Chem.,* 1997, vol. 16, 545-553
- SONNENSCHEIN, C. ; SOTO, A. M. *J. Steroid Biochem. Mol. Biol.,* 1998, vol. 65, 143-150
- COLBORN T. *Environ. Health Perspect.,* 1995, vol. 103, 135-136
- NEUBERT, D. *Regul. Toxicol. Pharmacol.,* 1997, vol. 26, 9-29
- DASTON, G. P. et al. *J. W. Reprod. Toxicol.,* 1997, vol. 11, 465-481
- SALOMONSSON, M. et al. *J. Steroid Biochem. Mol. Biol.,* 1994, vol. 50, 313-318
- KUIPER, G. G. J. M. et al. *Endocrinology,* 1997, vol. 138, 863-870
- TABIRA et al. *Eur. J. Biochem.,* 1999, vol. 262, 240-245
- BLAIR, R. M. et al. *Toxicol. Sci.,* 2000, vol. 54, 138-155
- BOLGER, R. et al. *Environ. Health Perspect.,* 1998, vol. 106, 551-557
- KURAMITZ, H. et al. *Anal. Chem.,* 2002, vol. 74, 533-538
- USAMI, M. et al. *Steroid Biochem. Mol. Biol.,* 2002, vol. 81, 47-55
- SOTO, A. M. et al. *Environ. Health Perspect.,* 1995, vol. 103 (7), 113-122
- BRONSTEIN, I. et al. *J. Anal. Biochem.,* 1994, vol. 219, 169-181
- GAIDO, K. W. et al. *Toxicol. Appl. Pharmcol.,* 1997, vol. 143, 205-213
- HERRY, D. M. et al. *Nature,* 1997, vol. 387, 733-736
- MAK, H. Y. et al. *Mol. Cell. Biol.,* 1999, vol. 19, 3895-3903
- WEATHERMAN, R. V. et al. *Mol Endocrinol.,* 2002, vol. 16, 487-496
- UEDA, H. et al. *J. Immunol. Methods,* 2003, vol. 279 (1-2), 209-18
- POLLOCK, B. A. ; HEIM, R. *Cell Biol.,* 1999, vol. 9, 57-60
- MOCHIZUKI, N. et al. *Nature,* 2001, vol. 411, 1065-1068
- SATO M. et al. *Nature Biotech.,* 2002, vol. 20, 287-294
- SASAKI, K. et al. *Biol. Chem.,* 2003, vol. 278, 30945-30951
- *Proc. Natl. Acad. Sci. USA,* 1980, vol. 77, 7380-7384
- BRZOZOWSKI, A. M. et al. *Nature,* 1997, vol. 389, 753-758
- SHIAU, A. K. et al. *Cell,* 1998, vol. 95, 927-937
- ROUTLEDGE, E. J. et al. *J. Biol. Chem.,* 2000, vol. 275, 35986-35993
- FANG, H. et al. *Chem. Res. Toxicol.,* 2001, vol. 14, 280-294
- JORDAN, V. C. et al. *Cancer Res,* 2001, vol. 61, 6619-6623

SEQUENCE LISTING

<110> Japan Science and Technology Agency

<120> Probe for detection of nuclear receptor agonist/antagonist and method for screening agonist and antagonist using the same

<130> 04F055PCT

<150> JP 2004-35678
<151> 2004-02-12

<160> 11

<170> PatentIn version 3.1

<210> 1
<211> 5
<212> PRT
<213> Unknown

<220>
<223> any amino acid

<220>
<221> MISC_FEATURE
<222> (2) .. (3)
<223> any amino acid

<400> 1

Leu Xaa Xaa Leu Leu
1                    5

<210> 2
<211> 11
<212> PRT
<213> Homo sapiens

<300>
<308> GeneBank/NP_671766
<309> 2003-12-22
<313> (687) .. (697)

<400> 2

His Lys Ile Leu His Arg Leu Leu Gln Glu Gly
1                    5                    10

<210> 3
<211> 239
<212> PRT
<213> Artificial

<220>
<223> synthesized polypeptide

<300>
<308> GeneBank/GAG11884
<309> 2003-05-21
<313> (1) .. (238)

<400> 3

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
            35                  40                  45

Cys Thr Thr Gly Lys Leu Pro Val Pro Trp Pro Thr Leu Val Thr Thr
        50                  55              · 60

Leu Thr Trp Gly Val Gln Cys Phe Ser Arg Tyr Pro Asp His Met Lys

65                   70                  75                  80

'

Gln His Asp Phe Phe Lys Ser Ala Met Pro Glu Gly Tyr Val Gln Glu

85                   90                  95

Arg Thr Ile Phe Phe Lys Asp Asp Gly Asn Tyr Lys Thr Arg Ala Glu

100                 105                 110

Val Lys Phe Glu Gly Asp Thr Leu Val Asn Arg Ile Glu Leu Lys Gly

115                 120                 125

Ile Asp Phe Lys Glu Asp Gly Asn Ile Leu Gly His Lys Leu Glu Tyr

130                 135                 140

Asn Tyr Ile Ser His Asn Val Tyr Ile Thr Ala Asp Lys Gln Lys Asn

145                 150                 155                 160

Gly Ile Lys Ala Asn Phe Lys Ile Arg His Asn Ile Glu Asp Gly Ser

165                 170                 175

Val Gln Leu Ala Asp His Tyr Gln Gln Asn Thr Pro Ile Gly Asp Gly

180                    185                    190


Pro Val Leu Leu Pro Asp Asn His Tyr Leu Ser Tyr Gln Ser Ala Leu
        195                    200                    205


Ser Lys Asp Pro Asn Glu Lys Arg Asp His Met Val Leu Leu Glu Phe
    210                    215                    220


Val Thr Ala Ala Gly Ile Thr Leu Gly Met Asp Glu Leu Tyr Lys
    225                    230                    235

<210> 4
<211> 248
<212> PRT
<213> unknown

<220>
<223> Artificial Sequence

<300>
<308> GeneBank/AAQ93355
<309> 2003-10-12
<313> (1) .. (238)

<400> 4

Met Val Ser Lys Gly Glu Glu Leu Phe Thr Gly Val Val Pro Ile Leu
1                5                      10                     15

Val Glu Leu Asp Gly Asp Val Asn Gly His Lys Phe Ser Val Ser Gly
                20                     25                     30

Glu Gly Glu Gly Asp Ala Thr Tyr Gly Lys Leu Thr Leu Lys Phe Ile
                35                     40                     45

Cys Thr Thr Gly Lys Leu Pro Val Pro Trp Pro Thr Leu Val Thr Thr
                50                     55                     60

Phe Gly Tyr Gly Leu Gln Cys Phe Ala Arg Tyr Pro Asp His Met Lys
65                     70                     75                     80

Gln His Asp Phe Phe Lys Ser Ala Met Pro Glu Gly Tyr Val Gln Glu
                85                     90                     95

Arg Thr Ile Phe Phe Lys Asp Asp Gly Asn Tyr Lys Thr Arg Ala Glu
                100                    105                    110

Val Lys Phe Glu Gly Asp Thr Leu Val Asn Arg Ile Glu Leu Lys Gly
115 120 125

Ile Asp Phe Lys Glu Asp Gly Asn Ile Leu Gly His Lys Leu Glu Tyr
130 135 140

Asn Tyr Asn Ser His Asn Val Tyr Ile Met Ala Asp Lys Gln Lys Asn
145 150 155 160

Gly Ile Lys Val Asn Phe Lys Ile Arg His Asn Ile Glu Asp Gly Ser
165 170 175

Val Gln Leu Ala Asp His Tyr Gln Gln Asn Thr Pro Ile Gly Asp Gly
180 185 190

Pro Val Leu Leu Pro Asp Asn His Tyr Leu Ser Tyr Gln Ser Ala Leu
195 200 205

Ser Lys Asp Pro Asn Glu Lys Arg Asp His Met Val Leu Leu Glu Phe
210 215 220

Val Thr Ala Ala Gly Ile Thr Leu Gly Met Asp Glu Leu Tyr Lys Ser
225               230               235               240

Gly Leu Arg Ser Thr Gly Ser Arg
245

<210> 5
<211> 246
<212> PRT
<213> Homo sapiens

<220>
<223> part of a eucaryotic protein

<300>
<308> GeneBank/NP_036821
<309> 2004-01-23
<313> (305) .. (550)

<400> 5

His Thr Lys Lys Asn Ser Pro Ala Leu Ser Leu Thr Ala Asp Gln Met
1               5               10               15

Val Ser Ala Leu Leu Asp Ala Glu Pro Pro Leu Ile Tyr Ser Glu Tyr
20 25 30

Asp Pro Ser Arg Pro Phe Ser Glu Ala Ser Met Met Gly Leu Leu Thr
35 40 45

Asn Leu Ala Asp Arg Glu Leu Val His Met Ile Asn Trp Ala Lys Arg
50 55 60

Val Pro Gly Phe Gly Asp Leu Asn Leu His Asp Gln Val His Leu Leu
65 70 75 80

Glu Cys Ala Trp Leu Glu Ile Leu Met Ile Gly Leu Val Trp Arg Ser
85 90 95

Met Glu His Pro Gly Lys Leu Leu Phe Ala Pro Asn Leu Leu Leu Asp
100 105 110

Arg Asn Gln Gly Lys Cys Val Glu Gly Met Val Glu Ile Phe Asp Met
115 120 125

Leu Leu Ala Thr Ser Ser Arg Phe Arg Met Met Asn Leu Gln Gly Glu
130             135             140

Glu Phe Val Cys Leu Lys Ser Ile Ile Leu Leu Asn Ser Gly Val Tyr
145             150             155             160

Thr Phe Leu Ser Ser Thr Leu Lys Ser Leu Glu Glu Lys Asp His Ile
                165             170             175

His Arg Val Leu Asp Lys Ile Asn Asp Thr Leu Ile His Leu Met Ala
                180             185             190

Lys Ala Gly Leu Thr Leu Gln Gln Gln His Arg Arg Leu Ala Gln Leu
                195             200             205

Leu Leu Ile Leu Ser His Ile Arg His Met Ser Asn Lys Gly Met Glu
210             215             220

His Leu Tyr Asn Met Lys Cys Lys Asn Val Val Pro Leu Tyr Asp Leu
225             230             235             240

Leu Leu Glu Met Leu Asp
                245

<210> 6
<211> 5
<212> PRT
<213> Artificial

<220>
<223> synthesized oligopeptide

<400> 6

Gly Gly Asn Gly Gly
1               5

<210> 7
<211> 11
<212> PRT
<213> Artificial

<220>
<223> synthesized oligopeptide

<400> 7

His Lys Ile Ala His Arg Ala Ala Gln Glu Gly
1               5               10

<210> 8
<211> 271
<212> PRT
<213> Homo sapiens

<220>
<223> part of a eucaryotic protein

<300>
<308> GenBank/NM_015869
<309> 1996-11-04
<313> (235) .. (505)

<400> 8

Glu Ser Ala Asp Leu Arg Ala Leu Ala Lys His Leu Tyr Asp Ser Tyr
1               5               10              15

Ile Lys Ser Phe Pro Leu Thr Lys Ala Lys Ala Arg Ala Ile Leu Thr
20              25              30

Gly Lys Thr Thr Asp Lys Ser Pro Phe Val Ile Tyr Asp Met Asn Ser
          35                40                45

Leu Met Met Gly Glu Asp Lys Ile Lys Phe Lys His Ile Thr Pro Leu
          50                55                60

Gln Glu Gln Ser Lys Glu Val Ala Ile Arg Ile Phe Gln Gly Cys Gln
65                70                75                80

Phe Arg Ser Val Glu Ala Val Gln Glu Ile Thr Glu Tyr Ala Lys Ser
                  85                90                95

Ile Pro Gly Phe Val Asn Leu Asp Leu Asn Asp Gln Val Thr Leu Leu
          100               105               110

Lys Tyr Gly Val His Glu Ile Ile Tyr Thr Met Leu Ala Ser Leu Met
          115               120               125

Asn Lys Asp Gly Val Leu Ile Ser Glu Gly Gln Gly Phe Met Thr Arg
          130               135               140

Glu Phe Leu Lys Ser Leu Arg Lys Pro Phe Gly Asp Phe Met Glu Pro
145          150          155          160

Lys Phe Glu Phe Ala Val Lys Phe Asn Ala Leu Glu Leu Asp Asp Ser
165          170          175

Asp Leu Ala Ile Phe Ile Ala Val Ile Ile Leu Ser Gly Asp Arg Pro
180          185          190

Gly Leu Leu Asn Val Lys Pro Ile Glu Asp Ile Gln Asp Asn Leu Leu
195          200          205

Gln Ala Leu Glu Leu Gln Leu Lys Leu Asn His Pro Glu Ser Ser Gln
210          215          220

Leu Phe Ala Lys Leu Leu Gln Lys Met Thr Asp Leu Arg Gln Ile Val
225          230          235          240

Thr Glu His Val Gln Leu Leu Gln Val Ile Lys Lys Thr Glu Thr Asp
245          250          255

Met Ser Leu His Pro Leu Leu Gln Glu Ile Tyr Lys Asp Leu Tyr
260          265          270

<210> 9

<211> 30
<212> PRT
<213> Artificial

<220>
<223> synthesized oligopeptide

<400> 9

Gly Gly Asn Gly Gly Gly Gly Asn Gly Gly Gly Gly Asn Gly Gly Gly
1               5                   10                  15

Gly Asn Gly Gly Gly Gly Asn Gly Gly Gly Gly Asn Gly Gly
        20                  25                  30

<210> 10
<211> 239
<212> PRT
<213> Homo sapiens

<220>
<223> part of a eucaryotic protein

<300>
<308> GenBank/M23263
<309> 2002-02-11
<313> (672) .. (910)

<400> 10

Phe Leu Asn Val Leu Glu Ala Ile Glu Pro Gly Val Val Cys Ala Gly
1               5                   10                  15

His Asp Asn Asn Gln Pro Asp Ser Phe Ala Ala Leu Leu Ser Ser Leu
                20                  25                  30

Asn Glu Leu Gly Glu Arg Gln Leu Val His Val Val Lys Trp Ala Lys
            35                  40                  45

Ala Leu Pro Gly Phe Arg Asn Leu His Val Asp Asp Gln Met Ala Val
        50                  55                  60

Ile Gln Tyr Ser Trp Met Gly Leu Met Val Phe Ala Met Gly Trp Arg
65                  70                  75                  80

Ser Phe Thr Asn Val Asn Ser Arg Met Leu Tyr Phe Ala Pro Asp Leu
                  85                  90                  95

Val Phe Asn Glu Tyr Arg Met His Lys Ser Arg Met Tyr Ser Gln Cys
                  100                 105                 110

Val Arg Met Arg His Leu Ser Gln Glu Phe Gly Trp Leu Gln Ile Thr
                  115                 120                 125

Pro Gln Glu Phe Leu Cys Met Lys Ala Leu Leu Leu Phe Ser Ile Ile
                  130                 135                 140

Pro Val Asp Gly Leu Lys Asn Gln Lys Phe Phe Asp Glu Leu Arg Met
                  145                 150                 155                 160

Asn Tyr Ile Lys Glu Leu Asp Arg Ile Ile Ala Cys Lys Arg Lys Asn
                  165                 170                 175

Pro Thr Ser Cys Ser Arg Arg Phe Tyr Gln Leu Thr Lys Leu Leu Asp
                  180                 185                 190

Ser Val Gln Pro Ile Ala Arg Glu Leu His Gln Phe Thr Phe Asp Leu

Leu Ile Lys Ser His Met Val Ser Val Asp Phe Pro Glu Met Met Ala

Glu Ile Ile Ser Val Gln Val Pro Lys Ile Leu Ser Gly Lys Val

<210> 11
<211> 15
<212> PRT
<213> Artificial

<220>
<223> synthesized oligopeptide

<400> 11

Gly Gly Asn Gly Gly Gly Gly Asn Gly Gly Gly Gly Asn Gly Gly